# EUROPEAN PATENT APPLICATION

(11) **EP 3 777 868 A1**
(43) Date of publication of application: **17.02.2021**
(21) Application number: 18911257.6
(22) Date of filing: 23.03.2018
(51) Int. Cl.: A61K 35/745, A61P 1/16, A61P 3/06, A61P 3/10, A61P 9/00, A61P 13/12, A61P 27/02, A61P 35/00, C12N 1/00

(54) **COMPOSITION FOR INFANTS FOR THE PREVENTION OF DISORDERS CAUSED BY HIGH BLOOD SUGAR IN CHILDHOOD AND BEYOND**

(71) Applicant: Morinaga Milk Industry Co., Ltd., Minato-ku Tokyo 108-8384 (JP)
(72) Inventor: EHARA, Tatsuya, Zama-shi, Kanagawa 252-8583 (JP); IZUMI, Hirohisa, Zama-shi, Kanagawa 252-8583 (JP); MATSUNAGA, Yutaka, Zama-shi, Kanagawa 252-8583 (JP); SHIMIZU, Takashi, Zama-shi, Kanagawa 252-8583 (JP)
(74) Representative: Vossius & Partner Patentanwälte Rechtsanwälte mbB
(86) International application number: PCT/JP2018/011922
(87) International publication number: WO 2019/180965

(57) **Abstract**

An object of the present invention is to provide a composition for infants and/or children for preventing a disease resulting from high blood sugar level in later childhood and thereafter, and a composition for infants and/or children for use in suppressing blood sugar level elevation in later childhood and thereafter. This object is achieved by a composition for infants and/or children for preventing a disease resulting from high blood sugar level in later childhood and thereafter, which contains a *Bifidobacterium* bacterium as an active ingredient, and a composition for infants and/or children for use in suppressing blood sugar level elevation in later childhood and thereafter, which contains a *Bifidobacterium* bacterium as an active ingredient.

SEQUENCE LISTING

<110>  MORINAGA MILK INDUSTRY CO., LTD.

<120>  A composition for infant to prevent disease derived from
       hyperglycemia during childhood or later

<130>  FP285-18044

<160>  4

<170>  PatentIn version 3.5

<210>  1
<211>  20
<212>  DNA
<213>  Artificial Sequence

<220>
<223>  primer 1

<400>  1
actcctacgg gaggcagcag                                                   20


<210>  2
<211>  17
<212>  DNA
<213>  Artificial Sequence

<220>
<223>  primer 2

<400>  2
attaccgcgg ctgctgg                                                      17


<210>  3
<211>  17
<212>  DNA
<213>  Artificial Sequence

<220>
<223>  primer 3

<400>  3
ctcctggaaa cgggtgg                                                      17


<210>  4
<211>  22
<212>  DNA
<213>  Artificial Sequence

<220>
<223>  primer 4

<400>  4
ggtgttcttc ccgatatcta ca                                                22

## Description

### Technical Field

The present invention relates to a composition for infants and/or children for preventing a disease resulting from high blood sugar level in later childhood and thereafter, and a composition for infants and/or children for use in suppressing elevation of blood sugar level in later childhood and thereafter.

More precisely, the present invention relates to a composition for infants and/or children for preventing a disease resulting from high blood sugar level in later childhood and thereafter, which contains a *Bifidobacterium* bacterium as an active ingredient, and a composition for infants and/or children used for suppressing elevation of blood sugar level in later childhood and thereafter, which contains a *Bifidobacterium* bacterium as an active ingredient.

### Background Art

In recent years, because of decrease in opportunities for excise, and westernization of eating habits, risks of onsets of obesity and metabolic syndrome are increasing. Obesity is caused by continuing a life with energy intake higher than energy expenditure in many cases. Obesity accompanied by superfluous accumulation of visceral fat (viscera fat obesity) and abnormalities in two or more of blood pressure, lipid value, and blood sugar level is defined as metabolic syndrome. In metabolic syndrome, abnormalities are caused in usual saccharometabolism and lipid metabolism in the body by superfluous accumulation of visceral fat, and possibilities of onsets of lifestyle-related diseases (for example, hypertension, hyperlipidemia, myocardial infarction, angina pectoris, diabetes, etc.) become high. Therefore, it is extremely important to prevent and improve obesity and metabolic syndrome by improving basal metabolism with maintaining health.

Hepatic diseases include alcoholic hepatic diseases caused by drinking, and non-alcoholic fatty liver diseases (NAFLDs) not caused by drinking, as well as viral hepatitis, liver cirrhosis, liver cancer, and so forth. The non-alcoholic liver diseases (NAFLDs) include non-alcoholic fatty liver (NAFL) and non-alcoholic steatohepatitis (NASH). If appropriate treatments are not given, conditions of alcoholic liver diseases and liver diseases caused by hepatitis virus infection are aggravated, and they develop into liver cirrhosis or liver cancer. If non-alcoholic fatty liver (NAFL) advances, it develops into non-alcoholic steatohepatitis (NASH), then the conditions are aggravated, and it develops into liver cirrhosis or liver cancer. It has also been reported that type II diabetes mellitus patients carry high risks of suffering from chronic liver diseases including non-alcoholic fatty liver diseases (NAFLDs) (Non-patent document 1).

It has so far been reported that, by administering a *Lactobacillus* bacterium such as *Lactobacillus rhamnosus* ATCC 53103, or a *Bifidobacterium* bacterium such as *Bifidobacterium longum* ATCC BAA-999 to a woman in the gestation period, or infant and/or child after birth to increase bifidobacteria in the intestines, and establish bifidobacterium-dominant intestinal bacterial flora, obesity of the infant and/or child can be prevented thereafter (Patent document 1).

However, it is not known that *Bifidobacterium* bacteria are effective for preventing a disease resulting from high blood sugar level in later childhood and thereafter for infants and/or children who have taken the bacteria (including those "who have been administered with them"), and suppressing elevation of blood sugar level in later childhood and thereafter for such infants and/or children.

### Prior art references

### Patent document

Patent document 1: US 2010/0111915 A1

### Non-patent document

Non-patent document 1: Arrese M., et al., Nat. Rev. Endocrinol., 6(12):660-1 (2010)

### Summary of the Invention

### Object to be Achieved by the Invention

An object of the present invention is to provide a composition for infants and/or children for preventing a disease resulting from high blood sugar level in later childhood and thereafter. Another object of the present invention is to provide a composition for infants and/or children for use in suppressing blood sugar level elevation in later childhood and thereafter, which contains a *Bifidobacterium* bacterium as an active ingredient.

### Means for Achieving the Object

The inventors of the present invention found that the *Bifidobacterium* bacteria prevent a disease resulting from high blood sugar level in later childhood and thereafter for infants and/or children who have taken the bacteria (including those "who have been administered with them") (the bacteria prevent a disease resulting from high blood sugar level in later childhood and thereafter in subjects who have taken the bacteria (including those "who have been administered with them") in their infant and/or child period), and came to accomplish the present invention. The inventors of the present invention also found that the *Bifidobacterium* bacteria are effective for suppressing blood sugar level elevation in later childhood and thereafter for infants and/or children who have taken the bacteria (including those "who have been administered with them") (the bacteria are effective for suppressing blood sugar level elevation in later childhood and thereafter in subjects who have taken the bacteria (including those "who have been administered with them") in their infant and/or child period), and came to accomplish the present invention.

That is, the present invention provides a composition for infants for preventing a disease resulting from high blood sugar level in later childhood and thereafter, which contains a *Bifidobacterium* bacterium as an active ingredient.

In a preferred embodiment of the composition, the disease resulting from high blood sugar level is hyperglycemia, hyperinsulinaemia, hyperlipidemia, diabetes, diabetic retinopathy, diabetic nephropathy, arteriosclerosis, myocardial infarction, non-alcoholic fatty liver, non-alcoholic steatohepatitis, liver cirrhosis, or liver cancer, and the liver cirrhosis and liver cancer are cirrhosis and liver cancer resulting from non-alcoholic fatty liver and/or non-alcoholic steatohepatitis.

The present invention also provides a composition for infants and/or children for use in suppressing blood sugar level elevation in later childhood and thereafter, which contains a *Bifidobacterium* bacterium as an active ingredient.

In a preferred embodiment of the composition, the infants and/or children are infants and/or children in a period of from birth to completion of weaning.

In a preferred embodiment of the composition, the composition does not aim at increase of the *Bifidobacterium* bacterium in the intestines.

In a preferred embodiment of the composition, the *Bifidobacterium* bacterium is *Bifidobacterium breve.*

In a preferred embodiment of the composition, the *Bifidobacterium breve* is *Bifidobacterium breve* M-16V (NITE BP-02622) .

In a preferred embodiment of the composition, the composition is a food or drink composition.

In a preferred embodiment of the composition, the composition is a pharmaceutical composition.

The present invention also provides use of a *Bifidobacterium* bacterium in manufacture of a composition for infants and/or children for preventing a disease resulting from high blood sugar level in later childhood and thereafter.

The present invention also provides a *Bifidobacterium* bacterium used for infants and/or children for preventing a disease resulting from high blood sugar level in later childhood and thereafter.

The present invention also provides use of a *Bifidobacterium* bacterium for infants and/or children for preventing a disease resulting from high blood sugar level in later childhood and thereafter.

The present invention also provides a method for preventing a disease resulting from high blood sugar level in later childhood of an infant and/or child and thereafter, which comprises the step of administering a *Bifidobacterium* bacterium to the infant and/or child.

### Effect of the Invention

According to the present invention, a composition for infants and/or children for preventing a disease resulting from high blood sugar level in later childhood and thereafter can be provided. According to the present invention, a composition for infants and/or children for use in suppressing blood sugar level elevation in later childhood and thereafter can also be provided.

### Modes for Carrying out the Invention

Hereafter, the present invention will be explained in detail.

The composition for infants and/or children of the present invention for preventing a disease resulting from high blood sugar level in later childhood and thereafter, and the composition for infants and/or children of the present invention for use in suppressing blood sugar level elevation in later childhood and thereafter contain a *Bifidobacterium* bacterium as an active ingredient. Hereafter, the compositions may be referred to as "the compositions of the present invention", and the bacterium may be referred to as "the bacterium of the present invention".

The compositions of the present invention may be a mixture, and it is not critical whether the ingredients of the compositions of the present invention are in a uniform state or non-uniform state.

The bacterium of the present invention as the active ingredient of the compositions of the present invention has an effect of preventing a disease resulting from high blood sugar level in later childhood and thereafter for an infant and/or child who has taken the bacterium (including infant and/or child "who has been administered with the bacterium" in this description). The bacterium of the present invention as the active ingredients of the compositions of the present invention also has an effect of suppressing blood sugar level elevation in later childhood and thereafter for an infant and/or child who has taken the bacterium (including infant and/or child "who has been administered with the bacterium" in this description).

The *Bifidobacterium* bacterium used in the present invention is a gram-positive strictly anaerobic bacillus.

The *Bifidobacterium* bacterium is not particularly limited, so long as, if it ingested by an infant and/or child, it can prevent a disease resulting from high blood sugar level of the infant and/or child in later childhood and thereafter, or it can suppress blood sugar level elevation of the infant and/or child in later childhood and thereafter. There can be used, for example, *Bifidobacterium longum* subsp. *longum, Bifidobacterium lactis, Bifidobacterium animalis, Bifidobacterium breve, Bifidobacterium longum* subsp. *infantis, Bifidobacterium adolescentis,* and so forth. *Bifidobacterium longum* subsp. *longum* may be abbreviated simply as *Bifidobacterium longum,* and *Bifidobacterium longum* subsp. *infantis* may be abbreviated simply as *Bifidobacterium infantis.*

For the present invention, *Bifidobacterium breve, Bifidobacterium longum* subsp. *longum,* and *Bifidobacterium longum* subsp. *infantis* are preferred.

Among them, *Bifidobacterium breve* FERM BP-11175, *Bifidobacterium breve* M-16V (NITE BP-02622), *Bifidobacterium longum* subsp. *longum* BB536 (NITE BP-02621), and *Bifidobacterium longum* subsp. *infantis* M-63 (NITE BP-02623) are more preferred.

The bacterium assigned the accession number of FERM BP-11175 was deposited at the independent administrative agency, National Institute of Advanced Industrial Science and Technology, International Patent Organism Depository (currently, independent administrative agency, National Institute of Technology and Evaluation, International Patent Organism Depositary, #120, 2-5-8 Kazusakamatari, Kisarazu-shi, Chiba-ken, 292-0818, Japan) on August 25, 2009 as an international deposit according to the provisions of the Budapest Treaty.

The bacterium assigned the accession number of NITE BP-02622 was deposited at the independent administrative agency, National Institute of Technology and Evaluation, NITE Patent Microorganism Depositary (#122, 2-5-8 Kazusakamatari, Kisarazu-shi, Chiba-ken, 292-0818, Japan) on January 26, 2018 as an international deposit according to the provisions of the Budapest Treaty.

The bacterium assigned the accession number of NITE BP-02621 was deposited at the independent administrative agency, National Institute of Technology and Evaluation, NITE Patent Microorganism Depositary (#122, 2-5-8 Kazusakamatari, Kisarazu-shi, Chiba-ken, 292-0818, Japan) on January 26, 2018 as an international deposit according to the provisions of the Budapest Treaty.

The bacterium assigned the accession number of NITE BP-02623 was deposited at the independent administrative agency, National Institute of Technology and Evaluation, NITE Patent Microorganism Depositary (#122, 2-5-8 Kazusakamatari, Kisarazu-shi, Chiba-ken, 292-0818, Japan) on January 26, 2018 as an international deposit according to the provisions of the Budapest Treaty.

The *Bifidobacterium* bacterium of the present invention is not limited to the aforementioned deposited strains, but may be a bacterium substantially equivalent to any of the deposited bacteria. The term bacterium substantially equivalent to any of the deposited bacteria means a bacterium of the same genus or species as that of the corresponding deposited bacterium, which can prevent a disease resulting from high blood sugar level in later childhood and thereafter for an infant and/or child, or can suppress blood sugar level elevation in later childhood and thereafter of the infant and/or child, if the infant and/or child has taken the bacterium, has a nucleotide sequence of the 16S rRNA gene showing a homology of 98% or higher, preferably 99% or higher, more preferably 100% or higher to the nucleotide sequence of the 16S rRNA gene of the corresponding deposited bacterium, and preferably shows the same bacteriological characteristics as those of the corresponding deposited bacterium in addition to the homology. The *Bifidobacterium* bacterium of the present invention may also be a bacterium bred from any of the deposited bacteria and bacteria substantially equivalent to them by mutation, gene recombination, selection of spontaneously mutated strains, or the like, so long as the effect of the present invention is not degraded.

The bacterium of the present invention may consist of bacterial cells or culture containing the cells. Although the bacterium of the present invention may consist of either one or both of live cells and dead cells, it preferably consists of live cells. The bacterium of the present invention may be subjected to various additional operations such as lyophilization after the culture, so long as the effect of the present invention is not degraded. The additional operations are preferably those allowing high survivability of live cells.

The bacterium of the present invention can be easily obtained by, for example, culturing the bacterium. The culture method is not particularly limited so long as the bacterium of the present invention can proliferate, and a method usually used for culture of *Bifidobacterium* bacteria (bifidobacteria) can be used with appropriate modifications as required. For example, culture temperature may be 25 to 50°C, preferably 35 to 40°C. Although the culture may be performed under aerobic conditions or anaerobic conditions, it is preferably performed under anaerobic conditions, and the culture can be performed, for example, with supplying anaerobic gas such as carbon dioxide. The culture may also be performed under microaerobic conditions, for example, as stationary liquid culture, or the like.

The medium for culturing the bacterium of the present invention is not particularly limited, and a medium usually used for culture of *Bifidobacterium* bacteria can be used with appropriate modifications as required. That is, as the carbon source, for example, saccharides such as galactose, glucose, fructose, mannose, cellobiose, maltose, lactose, sucrose, trehalose, starch, starch hydrolysate, and blackstrap molasses can be used according to the assimilation property of the bacterium. As the nitrogen source, for example, ammonia, ammonium salts such as ammonium sulfate, ammonium chloride, and ammonium nitrate, and nitrates can be used. As inorganic salts, sodium chloride, potassium chloride, potassium phosphate, magnesium sulfate, calcium chloride, calcium nitrate, manganese chloride, ferrous sulfate, and so forth can be used. Organic components such as peptone, soybean flour, degreased soybean meal, meat extract, and yeast extract may also be used. As already prepared medium, for example, the MRS medium can be preferably used.

Examples of the disease resulting from high blood sugar level referred to in the present invention include, for example, hyperglycemia, hyperinsulinaemia, hyperlipidemia, diabetes, diabetic retinopathy, diabetic nephropathy, arteriosclerosis, myocardial infarction, non-alcoholic fatty liver (NAFL), non-alcoholic steatohepatitis (NASH), liver cirrhosis and liver cancer resulting from the non-alcoholic fatty liver and/or non-alcoholic steatohepatitis mentioned above, and so forth. Non-alcoholic steatohepatitis (NASH) may also be referred to as non-alcoholic fatty hepatitis. Non-alcoholic fatty liver (NAFL) and non-alcoholic steatohepatitis (NASH) are also collectively referred to as non-alcoholic fatty liver disease (NAFLD). Diabetes includes type I diabetes mellitus, type II diabetes mellitus, pregnancy diabetes mellitus, and diabetes mellitus caused by another specific mechanism or disease.

When a subject suffers from hyperglycemia, hyperinsulinaemia, hyperlipidemia, diabetes, diabetic retinopathy, diabetic nephropathy, arteriosclerosis, myocardial infarction, or the like, blood sugar level increased after administration of glucose does easily decrease. Concerning this phenomenon, a subject who took the bacterium of the present invention in the infant and/or child period, and has continuously taken high fat diet in later childhood and thereafter after growth shows smaller blood sugar level, the blood sugar level is elevated after administration of glucose, compared with that of such a subject who did not take the bacterium of the present invention in the infant and/or child period, for example, in such an insulin tolerance test as described in the examples mentioned later. Namely, the bacterium of the present invention suppresses elevation of blood sugar level, and maintains the blood sugar level to be normal, or minimize the degree of abnormality of the blood sugar level in later childhood and thereafter for an infant and/or child who took the bacterium after growth, and therefore it can prevent hyperglycemia, hyperinsulinaemia, hyperlipidemia, diabetes, diabetic retinopathy, diabetic nephropathy, arteriosclerosis, myocardial infarction, and so forth in later childhood and thereafter.

When a subject suffers from hyperglycemia, hyperinsulinaemia, hyperlipidemia, diabetes, diabetic retinopathy, diabetic nephropathy, arteriosclerosis, myocardial infarction, or the like, there is observed a condition called insulin resistance, namely, in spite of the existence of insulin in blood, the blood sugar level does not decrease, because the action of insulin is not fully exerted. Concerning this phenomenon, a subject who took the bacterium of the present invention in the infant and/or child period, and has continuously taken high fat diet in later childhood and thereafter after growth shows smaller blood sugar level, the blood sugar level is decreased after administration of insulin, compared with that of such a subject who did not take the bacterium in the infant and/or child period, for example, in such an insulin tolerance test as described in the examples mentioned later. Namely, the bacterium makes the action of insulin be normally exerted, or minimize the degree of insulin resistance of an infant and/or child who took the bacterium of the present invention in later childhood and thereafter after growth, and therefore it can prevent hyperglycemia, hyperinsulinaemia, hyperlipidemia, diabetes, diabetic retinopathy, diabetic nephropathy, arteriosclerosis, myocardial infarction, and so forth in later childhood and thereafter by suppressing elevation of blood sugar level, and maintaining the blood sugar level to be normal, or minimize the degree of abnormality of the blood sugar level.

When a subject shows at least one of the conditions that the blood sugar level elevated after administration of glucose does not easily decrease, and that the subject shows insulin resistance, the subject is judged to suffer from hyperglycemia, hyperinsulinaemia, hyperlipidemia, diabetes, diabetic retinopathy, diabetic nephropathy, arteriosclerosis, myocardial infarction, or the like.

When a subject suffers from non-alcoholic fatty liver (NAFL), or non-alcoholic steatohepatitis (NASH) resulting from high blood sugar level; or liver cirrhosis or liver cancer resulting from the non-alcoholic fatty liver and/or non-alcoholic steatohepatitis mentioned above, the liver weight per unit body weight increases. Concerning this phenomenon, a subject who took the bacterium of the present invention in the infant and/or child period, and has continuously taken high fat diet in later childhood and thereafter after growth shows a smaller liver weight per unit body weight compared with that of such a subject who did not take the bacterium in the infant and/or child period, for example, in such a liver weight measurement test as described in the example mentioned later. Namely, the bacterium of the present invention suppresses elevation of blood sugar level, and maintains the blood sugar level to be normal, or minimize the degree of abnormality of the blood sugar level in later childhood and thereafter after growth of an infant and/or child who took the bacterium, and therefore it can prevent non-alcoholic fatty liver (NAFL), or non-alcoholic steatohepatitis (NASH) resulting from high blood sugar level; or liver cirrhosis or liver cancer resulting from the non-alcoholic fatty liver and/or non-alcoholic steatohepatitis mentioned above in later childhood and thereafter by suppressing increase of liver weight per unit body weight, or minimize increase of liver weight per unit body weight.

Another aspect of the present invention is a composition for infants and/or children for use in suppressing blood sugar level elevation in later childhood and thereafter. As described above, the bacterium of the present invention suppresses elevation of the blood sugar level, and maintains the blood sugar level to be normal, or minimize the degree of abnormality of the blood sugar level in later childhood of an infant and/or child who took the bacterium and thereafter. The bacterium also makes the action of insulin be normally exerted, or minimize the degree of insulin resistance in later childhood and thereafter after growth in an infant and/or child who took the bacterium. That is, the blood sugar level elevation-suppressing action referred to in the present invention is an action for making blood sugar level of a subject who took the bacterium of the present invention in the infant and/or child period to be lower than that observed without taking the bacterium of the present invention in the infant and/or child period in later childhood and thereafter. The latter blood sugar level is preferably that to be observed with continuously taking high fat diet in later childhood and thereafter as described above. As for degree of the suppression of blood sugar level elevation, blood sugar level observed in later childhood and thereafter after growth for a subject who took the bacterium in the infant and/or child period is preferably 90% or lower, more preferably 85% or lower, of blood sugar level to be observed in the later childhood and thereafter after growth, without taking the bacterium in the infant and/or child period.

The object of the use of the compositions of the present invention is infant and/or child of mammal. Type of the mammal is not limited, and examples include, for example, human, cow and bull, sheep, goat, pig, dog, cat, horse, and so forth. The object is preferably human.

In the following descriptions, stages of mammal are defined for human as an example the mammal. As for stages of mammals other than human, the defined stages can be converted into equivalent stages of a corresponding mammal other than human. When the object is human, the term "infant and/or child" used in the present invention means infant and/or child, and the infant may include a neonate. The term "infant" used for the present invention means a person in infant period, and refers to a person after the birth and younger than 1 year old (until the day before the day on which the person becomes 1 year old). The "infant period" is defined to refer to a stage where the major nutrition source is milk such as breast milk. The term "child" used for the present invention refers to a person in a period of from the day on which the person becomes 1 year old to the day before entering later childhood, preferably a person in a period of from the day on which the person becomes 1 year old to the day before the earlier day between the day on which the person becomes 6 years old and the day of entering elementary school.

The term "neonate" used for the present invention refers to an infant after birth and younger than 28 days old (up to the day before the day on which the infant becomes 28 days old). The "neonate" may be a "premature infant (born after a gestation period of 22 weeks or longer and shorter than 37 weeks)", or "low birth weight infant (neonate born with a birth weight lower than 2500 g)". The "premature infant" and "low birth weight infant" may be referred to as "immature infant".

The term "later childhood and thereafter" used for the present invention refers to "a period of the later childhood and a period thereafter", preferably the earlier day between the day on which a person becomes 6 years old and the day of entering elementary school, and a period thereafter.

In the examples mentioned later, results of experiments performed by using mice are shown. The correspondence of the stages of human and stages of mouse is as follows.

In the present invention, human "infant" corresponds to mouse after birth and up to 21 days of old.

In the present invention, human "child" corresponds to mouse of from 22 days old to 28 days old.

In the present invention, "later childhood and thereafter" of human corresponds to the 29th day after birth and a period thereafter for mouse.

The infant and/or child for whom the composition of the present invention is used is preferably an infant and/or child in a period of from the birth to the day before the earlier day between the day on which infant and/or child becomes 6 years old and the day of entering elementary school, more preferably an infant and/or child in a period of from the birth to the completion of weaning (the last day of weaning period), still more preferably an infant.

The term "weaning period" used in of the present invention means a period in which the diet is shifted from milk nutrition such as formulated milk powder for infants and mother's milk to child foods, and although it may be different among individuals, specifically, it is preferably a period of from the day on which the infant and/or child becomes 5 months old to the day on which the infant and/or child becomes 1.5 years old, more preferably a period of from the day on which the infant and/or child becomes 6 months old to the day on which the infant and/or child becomes 1 year old. Therefore, the time of "completion of weaning (the last day of weaning period)" mentioned in the present invention is preferably the day on which the infant and/or child becomes 1.5 years old, more preferably the day on which the infant and/or child becomes 1 year old.

In the present invention, human "weaning period" corresponds to a period up to about 18 to 28 days after the birth, preferably about 21 to 24 days after the birth, of mouse.

The compositions of the present invention preferably contain a prebiotic. Prebiotics are indigestible food ingredients that selectively change growth and activity of particular bacteria in the large intestines, thereby advantageously influence the host, and improve health of the host.

Although such a prebiotic is not particularly limited so long as it can promote the effect of the present invention when it is ingested by a mammal together with the bacterium of the present invention, for example, lactulose, raffinose, galactooligosaccharides, fructooligosaccharides, soybean oligosaccharides, lacto-fructo-oligosaccharides, xylooligosaccharides, isomaloligosaccharides, coffee bean mannooligosaccharides, gluconate, polydextrose, inulin and so forth are preferred. Among these, lactulose, raffinose, galactooligosaccharides, and so forth are more preferred, and lactulose, raffinose, and galactooligosaccharide are still more preferred.

Lactulose is a disaccharide consisting of fructose and galactose (4-O-β-D-galactopyranosyl-D-fructose, Gal β1-4 Fru), and can be produced by a known method, for example, the methods described in Japanese Patent Laid-open No. H03-169888, and Japanese Patent Laid-open No. H06-228179. As lactulose, commercial products (for example, those produced by Morinaga Milk Industry Co., Ltd., etc.) can also be used.

Raffinose is a trisaccharide consisting of each one of fructose, galactose, and glucose (β-D-fructofuranosyl-α-D-galactopyranosyl-(1-6)-α-D-glucopyranoside, Gal α1-6 Glc α1-2β Fru), and can be produce by a known method, for example, the method described in "Techniques for Effective Use of Food New Materials, Series No. 6, "Raffinose", page 2, Japan Confectionery and Innovative Food Ingredients Research Center, 1996". As raffinose, commercial products (for example, those produced by Nippon Beet Sugar Mfg. Co., Ltd., etc.) can also be used.

Galactosaccharide (GOS) is an oligosaccharide having a structure represented as Gal-(Gal)ₙ-Glc (n is 1 to 3, linkage is β-1,4 linkage or β-1,6 linkage), or a mixture of such oligosaccharides. Galactosaccharides are industrially produced by a transfer reaction catalyzed by a β-galactosidase using lactose as a raw material, and the main ingredient thereof is 4'-galactosyllactose (4'-GL), which is a trisaccharide consisting of lactose and one galactose linked to the non-reduced end of lactose. As the galactooligosaccharide, commercial products (for example, those produced by Yakult Pharmaceutical Industry Co., Ltd., etc.) can also be used. The galactooligosaccharide may consist of one kind of galactooligosaccharide, or two or more kinds of galactooligosaccharides.

The compositions of the present invention are preferably a composition that does not aim at increase in *Bifidobacterium* bacteria in the intestine. More precisely, The compositions of the present invention are preferably a composition that does not aim at increasing *Bifidobacterium* bacteria in the intestine by the bacterium and/or a prebiotic. The intestine is preferably intestine of an infant and/or child who took the composition of the present invention in the infant and/or child period.

An embodiment of the compositions of the present invention that aims at increase in *Bifidobacterium* bacteria in the intestine is preferably excluded from the scope of the present invention. An embodiment of use of the compositions of the present invention that aims at increase in *Bifidobacterium* bacteria in the intestine is also preferably excluded from the scope of the present invention.

The composition of the present invention can be used as a food or drink composition, or a pharmaceutical composition. For example, a food or drink composition for infants and/or children for preventing a disease resulting from high blood sugar level in later childhood and thereafter, and a pharmaceutical composition for infants and/or children for preventing a disease resulting from high blood sugar level in later childhood and thereafter can be provided. There can also be provided, for example, a food or drink composition for infants and/or children for use in suppressing blood sugar level elevation in later childhood and thereafter, and a pharmaceutical composition for infants and/or children for use in suppressing blood sugar level elevation in later childhood and thereafter. These food or drink compositions and pharmaceutical compositions may be henceforth correctively referred to as "food or drink composition of the present invention", and "pharmaceutical composition of the present invention", respectively.

The food or drink composition of the present invention is not particularly limited so long as it contains the bacterium of the present invention. Form of the food or drink composition is not particularly limited, and it may be in the form of liquid, paste, gellated solid, powder, or the like, and may be a food or drink. Examples include, for example, tablet confectioneries, and liquid diets, as well as, for example, flour products such as bread, macaroni, spaghetti, noodles, cake mix, fry powder and bread crumbs; ready-to-eat foods such as instant noodles, pot noodles, retort and cooked foods, canned cooking, foods for microwave heating, instant soup and stew, instant miso soup and Japanese clear soup, canned soup, freeze-dried foods, and other ready-to-eat foods; processed agricultural products such as canned agricultural products, canned fruits, jams and marmalades, pickles, cooked beans, dry agricultural products, and cereals (processed grain products); processed marine products such as canned marine products, fish ham and sausages, seafood paste products, marine dainties, and tsukudani (marine products boiled in soy source); processed livestock products such as canned livestock products and pastes, and livestock meat ham and sausages; milks and dairy products such as processed milk, milk drinks, yoghurts, lactic acid drinks, cheese, ice creams, modified milk powders, creams, and other dairy products; oils and fats such as butter, margarines, and vegetable oils; basic seasoning such as soy sauce, miso, sauces, processed tomato seasoning, mirin, and vinegars; complex seasonings and foods such as cooking mix, curry powder or roux, sauces for dipping, dressings, noodle sauces, spices, and other complex seasonings; frozen foods such as frozen food materials, semi-cooked frozen foods, and cooked frozen foods; confectioneries such as caramel candies, candies, gummi candies, chewing gums, chocolates, cookies, biscuits, cakes, pies, snacks, crackers, Japanese sweets, rice confectioneries, bean confectioneries, dessert pastries, jellies, and other confectioneries; beverages such as carbonated drinks, natural fruit juices, fruit juice drinks, fruit juice soft drinks, fruit pulp drinks, fruit drinks with fruit pulp, vegetable based drinks, soy milk, soy milk drinks, coffee drinks, tea drinks, powdered drinks, concentrated drinks, sports drinks, nutritional beverage, alcoholic drinks, and other beverages; other commercial foods such as baby foods, rice seasonings, and seaweed seasonings for boiled rice soaked with tea; formulated milk powder for infants and/or children; enteral nutrients; foods for special dietary uses, foods with health claims (foods for specified health use, foods with nutrient function claims, food with function claims); nutritional supplements, and so forth.

The food or drink composition of the present invention may be a supplement, for example, a supplement in the form of tablet. If the food or drink composition of the present invention is a supplement, the bacterium of the present invention can be taken without being influenced by other foods for daily amount of meals and ingested calories.

The food or drink composition of the present invention can be produced by adding the bacterium of the present invention to a raw material of usual foods or drinks, and can be produced in the same manner as those for usual foods or drinks except that the bacterium of the present invention is added. The bacterium of the present invention may be added at any stage of the manufacturing process of the food or drink composition. The food or drink composition may also be produced through a fermentation process advanced by the bacterium of the present invention. Examples of such a food or drink composition include lactic acid bacteria beverages, fermented milks, and so forth.

As the raw material of the food or drink composition, raw materials used for producing usual drinks and foods can be used. The produced food or drink composition can be orally taken.

The food or drink composition of the present invention may be a raw material for production of food or drink composition, or a material to be added to food or drink composition in the course of the food or drink composition production process or after the production, such as food additives. For example, the bacterium of the present invention can be used as a starter for production of fermented milk. The bacterium of the present invention can also be added to a produced fermented milk afterward.

Although content of the bacterium of the present invention in the food or drink composition of the present invention is appropriately determined according to the form of the food or drink composition, it is usually preferably in the range of 1 x 10⁴ to 1 x 10¹³ cfu/g, or 1 x 10⁴ to 1 x 10¹³ cfu/ml, more preferably in the range of 1 x 10⁵ to 1 x 10¹² cfu/g, or 1 x 10⁵ to 1 x 10¹² cfu/ml, still more preferably in the range of 1 x 10⁶ to 1 x 10¹⁰ cfu/g, or 1 x 10⁶ to 1 x 10¹⁰ cfu/ml, further preferably in the range of 1 x 10⁶ to 1 x 10⁸ cfu/g, or 1 x 10⁶ to 1 x 10⁸ cfu/ml, in terms of the total amount in the food or drink composition. The unit "cfu" represents colony forming unit. When the bacterium consists of dead cells, cfu/g or cfu/ml can be read as number of cells/g, or number of cells/ml.

When the food or drink composition of the present invention contains a prebiotic, the total amount of the bacterium of the present invention is preferably in the range of 1 x 10⁶ to 1 x 10¹² cfu, more preferably 1 x 10⁷ to 1 x 10¹² cfu, still more preferably 1 x 10⁸ to 1 x 10¹² cfu, per 1 g of the total amount of the prebiotic. When the prebiotic consists of lactulose, raffinose, and galactooligosaccharide, weight ratios of these are preferably 1 to 9:1 to 9:1 to 9, more preferably 2 to 8:2 to 8:2 to 8, still more preferably 3 to 7:3 to 7:3 to 7.

The food or drink composition of the present invention may be independently taken, or may be taken together with another food or drink composition, a food or drink, or a pharmaceutical composition or a drug. For example, it may be taken together with another food or drink composition, food or drink, pharmaceutical composition, drug, or the like for infants and/or children for preventing a disease resulting from high blood sugar level in later childhood and thereafter. It may also taken together with, for example, another food or drink composition, food or drink, pharmaceutical composition, drug, or the like for infants and/or children for use in suppressing blood sugar level elevation in later childhood and thereafter.

The food or drink composition of the present invention can be sold as a food or drink composition, or a food or drink with an indication of use that the composition is for infants and/or children, and for preventing a disease resulting from high blood sugar level in later childhood and thereafter after growth. Of course, any other indication representing the use for infants and/or children, and secondary effect provided by prevention of a disease resulting from high blood sugar level in later childhood and thereafter after growth can also be used.

The food or drink composition of the present invention can also be sold as a food or drink composition, or a food or drink with an indication of use that the composition is for infants and/or children, and for suppression of blood sugar level elevation in later childhood and thereafter after growth. Of course, any other indication representing the use that the composition is for infants and/or children, and secondary effect provided by suppression of blood sugar level elevation in later childhood and thereafter after growth can also be used.

Of course, the wording of the indication is not limited to wordings directly representing prevention of a disease resulting from high blood sugar level, or suppression of blood sugar level, and other wordings indicating effect for prevention, treatment, and/or improvement of a disease resulting from high blood sugar level and various related diseases, or effect for prevention, treatment, and/or improvement of blood sugar level elevation also fall within the scope of the present invention. Examples of such wordings include, for example, indications based on various uses that make consumers recognize the use for prevention, treatment, and/or improvement of a disease resulting from high blood sugar level and various related diseases and conditions, or for prevention, treatment, and/or improvement of blood sugar level elevation, such as "For persons who want to suppress blood sugar level", "For persons who concern about blood sugar level", and "For persons who want mild absorption of saccharides".

The food or drink composition of the present invention can also be provided and sold as a food or drink composition or a food or drink with an indication of use as probiotics etc. (including use for health). The food or drink composition of the present invention can also be provided and sold with an indication of target persons of ingestion, such as "Persons who want life of living with bifidobacteria", "Persons who want to improve intestinal environment", "Persons who want to get good conditions of intestines", and "Persons who want to form good intestinal environment".

The aforementioned term "indication" includes all actions for informing consumers the aforementioned use, and any indications reminding or analogizing the aforementioned use fall within the scope of the "indication" of the present invention regardless of purpose, content, objective article, medium etc. of the indication. However, the indication is preferably made with an expression that allows consumers to directly recognize the aforementioned use.

Specific examples include actions of indicating the aforementioned use on goods or packages of goods relating to the food or drink composition, or the food or drink of the present invention, actions of assigning, delivering, displaying for the purpose of assigning or delivering, or importing such goods or packages of goods on which the aforementioned use is indicated, displaying or distributing advertisements, price lists or business papers relating to the goods on which the aforementioned use is indicated, or providing information including those as contents with indicating the aforementioned use by an electromagnetic method (Internet etc.), and so forth. Indications on packages, containers, catalogues, pamphlets, advertisement materials used at the sales spots such as POPs, and other papers are especially preferred.

The indication is preferably an indication approved by the administration etc. (for example, an indication in a form based on an approval, which is qualified on the basis of any of various legal systems provided by the administration). Examples of such an indication include, for example, indications as food with health claims, or the like, more specifically, food with health claims, health food, functional food, enteric nutritive food, food for special dietary uses, food with nutrient function claims, quasi-drug, or the like, as well as indications approved by the Consumer Affairs Agency, for example, indications approved on the basis of the systems of food for specified health uses, food with nutrient function claims, food with function claims, and similar systems. Examples of the latter include indications as food for specified health uses, indications as food for specified health uses with qualified health claims, indications of influence on body structures and functions, indications of reduction of disease risk claims, indications of functional claims based on scientific evidence, and so forth, and more precisely, typical examples include indications as food for specified health uses (especially indications of use for health) provided in the Cabinet Office Ordinance concerning approval of indications of special dietary uses provided by the Health Promotion Law, and others (Cabinet Office Ordinance No. 57, August 31, 2009), and similar indications.

The pharmaceutical composition of the present invention is not particularly limited so long as it contains the bacterium of the present invention. As the pharmaceutical composition of the present invention, the bacterium of the present invention may be used as it is, or a preparation prepared from a mixture of the bacterium of the present invention and a physiologically acceptable liquid or solid pharmaceutical carrier may also be used.

Dosage form of the pharmaceutical composition of the present invention is not particularly limited, and specific examples include tablet (including sugar-coated tablet, enteric coated tablet, and buccal tablet), powder, capsule (including enteric capsule and soft capsule), granule (including coated granule), pill, troche, liposome-enclosed agent, solution, and pharmaceutically acceptable sustained release preparations of these. For preparation of the composition in the dosage form, there can be used additives widely used for usual oral drugs as ingredients of pharmaceutical preparations, such as carrier, excipient, binder, disintegrating agent, lubricant, stabilizer, corrigent, diluent, surfactant, and solvent. So long as the effect of the present invention is not degraded, the bacterium of the present invention and a known drug or pharmaceutical composition for infants and/or children for preventing a disease resulting from high blood sugar level in later childhood and thereafter, or such a drug or pharmaceutical composition to be found in the future may be used together. So long as the effect of the present invention is not degraded, the bacterium of the present invention and a known drug or pharmaceutical composition for infants and/or children for suppressing blood sugar level elevation in later childhood and thereafter, or such a drug or pharmaceutical composition to be found in the future may be used together.

As the carrier for pharmaceutical preparations, various organic or inorganic carriers can be used depending on the dosage form. Examples of the carrier used for solid preparations include, for example, excipient, binder, disintegrating agent, lubricant, stabilizer, corrigent, and so forth.

Examples of the excipient include, for example, saccharides and derivatives thereof such as lactose, sucrose, glucose, mannite, and sorbit; starch and derivatives thereof such as corn starch, potato starch, pregelatinized starch, dextrin, and carboxymethyl starch; crystalline cellulose, cellulose derivatives such as hydroxypropylcellulose, hydroxypropylmethylcellulose, carboxymethylcellulose, and carboxymethylcellulose calcium, etc.; gum arabic; dextran; pullulan; light silicic anhydride, and silicate and derivatives thereof such as synthetic aluminum silicate, and magnesium metasilicate aluminate; phosphate and derivatives thereof such as calcium phosphate; carbonate and derivatives thereof such as calcium carbonate; sulfate and derivatives thereof such as calcium sulfate, and so forth.

Examples of the binder include, for example, besides the aforementioned excipients, gelatin; polyvinylpyrrolidone; macrogol, and so forth.

Examples of the disintegrating agent include, for example, besides the aforementioned excipients, chemically modified starch or cellulose derivatives such as croscarmellose sodium, carboxymethyl starch sodium, and crosslinked polyvinylpyrrolidone, and so forth.

Examples of the lubricant include, for example, talc; stearic acid; stearic acid metal salts such as calcium stearate, and magnesium stearate; colloidal silica; waxes such as bee gum and spermaceti; boric acid; glycol; carboxylic acid such as fumaric acid and adipic acid; sodium carboxylate such as sodium benzoate; sulfate such as sodium sulfate; leucine; laurylsulfate such as sodium laurylsulfate and magnesium laurylsulfate; silicic acid such as silicic anhydride and silicic acid hydrate; starch derivatives, and so forth.

Examples of the stabilizer include, for example, p-oxybenzoic acid esters such as methylparaben and propylparaben; alcohols such as chlorobutanol, benzyl alcohol, and phenylethyl alcohol; benzalkonium chloride; acetic anhydride; sorbic acid, and so forth.

Examples of the corrigent include, for example, sweeteners, acidulants, perfumes, and so forth.

Examples of the carrier used for liquid preparations for oral administration include solvents such as water, corrigents, and so forth.

Content of the bacterium of the present invention in the pharmaceutical composition of the present invention can be appropriately determined depending on dosage form, scheme for use, age, sex, type and severity of disease, symptoms, and conditions of subject, other conditions, and so forth, and usual range and preferred range thereof are the same as those for the aforementioned food or drink composition of the present invention. When the pharmaceutical composition of the present invention contains a prebiotic, the total amount of the bacterium of the present invention to the total amount of the prebiotic of 1 g is the same as that for the aforementioned food or drink composition of the present invention. When the prebiotic consists of lactulose, raffinose, and galactooligosaccharide, weight ratios of these are also the same as those for the aforementioned food or drink composition of the present invention.

The dosage form of the pharmaceutical composition of the present invention is preferably determined according to the form of the preparation, age, sex, and other conditions of infants and/or children, and so forth. In any case, the pharmaceutical composition of the present invention can be administered once a day, or two or more times a day as divided portions, or may be administered once every several days or several weeks.

The pharmaceutical composition of the present invention may be administered independently, or may be administered together with another pharmaceutical composition, drug, food or drink composition, or food or drink. For example, it may be taken together with another pharmaceutical composition, drug, food or drink composition, food or drink, or the like for infants and/or children for preventing a disease resulting from high blood sugar level in later childhood and thereafter. It may also be taken together with, for example, another pharmaceutical composition, drug, food or drink composition, food or drink, or the like for infants and/or children for use in suppressing blood sugar level elevation in later childhood and thereafter.

Another aspect of the present invention is use of a *Bifidobacterium* bacterium in manufacture of a composition for infants and/or children for preventing a disease resulting from high blood sugar level in later childhood and thereafter.

Still another aspect of the present invention is a *Bifidobacterium* bacterium used for infants and/or children for preventing a disease resulting from high blood sugar level in later childhood and thereafter.

Still another aspect of the present invention is use of a *Bifidobacterium* bacterium for infants and/or children for preventing a disease resulting from high blood sugar level in later childhood and thereafter.

Still another aspect of the present invention is a method for preventing a disease resulting from high blood sugar level in later childhood and thereafter, which comprises the step of administering a *Bifidobacterium* bacterium to an infant and/or child, or the step of administering the composition of the present invention to an infant and/or child.

Still another aspect of the present invention is use of a *Bifidobacterium* bacterium in manufacture of a composition for infants and/or children for suppressing blood sugar level elevation in later childhood and thereafter.

Still another aspect of the present invention is a *Bifidobacterium* bacterium used for infants and/or children for use in suppressing blood sugar level elevation in later childhood and thereafter.

Still another aspect of the present invention is use of a *Bifidobacterium* bacterium for infants or children for suppressing blood sugar level elevation in later childhood and thereafter.

Still another aspect of the present invention is a method for suppression of blood sugar level elevation in later childhood and thereafter, which comprises the step of administering a *Bifidobacterium* bacterium to an infant and/or child, or the step of administering the composition of the present invention to an infant and/or child.

### Examples

Hereafter, the present invention will be still more specifically explained with reference to examples. However, the present invention is not limited to these examples.

### [Example 1]

Effect of *Bifidobacterium breve* M-16V (NITE BP-02622) on blood sugar level after administration of insulin, blood sugar level after administration of glucose, and liver weight per unit body weight in later childhood and thereafter for infant and/or child who took the bacterium

Cell powder of *Bifidobacterium breve* M-16V (NITE BP-02622) diluted in starch (2.4 x 10¹¹ cfu/g) was suspended in physiological saline to prepare a bifidobacterium suspension of 2.5 x 10⁹ cfu/ml.

Lactulose (Morinaga Milk Industry Co., Ltd.), raffinose (product name is Nitten Raffinose, Nippon Beet Sugar Mfg. Co., Ltd.), and a galactooligosaccharide were mixed and dissolved in distilled water at a weight ratio of 1:1:1 to prepare an oligosaccharide stock solution of a final total concentration of 250 mg/ml. The galactooligosaccharide was obtained by removing monosaccharides and disaccharides from a commercial product (product name is Oligomate 55N, Yakult Pharmaceutical Industry Co., Ltd.), and contained about 65% by weight of Galβ1-4Galβ1-4Glc (4'-galactosyllactose) and about 15% by weight of Galβ1-6Galβ1-4Glc (6'-galactosyllactose).

Physiological saline, the aforementioned bifidobacterium suspension diluted 5 times with physiological saline (final concentration of bifidobacterium was 5 x 10⁸ cfu/ml), a mixture of physiological saline and the oligosacharide stock solution at a volume ratio of 1:4 (oligosaccharide final concentration was 200 mg/ml), and a mixture of the bifidobacterium suspension and the oligosacharide stock solution at a volume ratio of 1:4 (bifidobacterium final concentration was 5 x 10⁸ cfu/ml, oligosaccharide final concentration was 200 mg/ml) were used as administration samples for the groups A to D mentioned below, respectively.

C57BL/6J male mice of 2 days old after birth and mother animals thereof were purchased from Japan SLC. The infant mice were allowed to freely take mother's milk of the mother animals. The mice were divided into the following four groups A to D at 5 days old so that body weight of the mice of the groups should be equivalent.
A: Vehicle group (physiological saline was administered)
B: *Bifidobacterium* group (diluted bifidobacterium suspension was administered)
C: Oligosaccharide group (oligosaccharide solution was administered)
D: Oligosaccharide + bifidobacterium group (mixture of oligosaccharide and bifidobacterium was administered)

To the infant mice of the groups, each administration sample was administered once every day in a volume of 100 µl for a period of from 6 days old to 20 days old after birth. That is, there were administered 5 x 10⁷ cfu of *Bifidobacterium breve* M-16V (NITE BP-02622) to the mice of the group B, 20 mg of oligosaccharides to the mice of the group C, and 20 mg of oligosaccharides and 5 x 10⁷ cfu of *Bifidobacterium breve* M-16V (NITE BP-02622) to the mice of the group D, per one time of administration.

After weaning these mice at 21 days old, they were fed with usual feed up to 28 days old, and fed with high fat feed from the day of 29 days old. However, a part of the mice of the group A were continuously fed with usual feed, and these mice were considered as a reference group.

During the high fat feed loading period, an insulin tolerance test and an oral carbohydrate tolerance test were performed. The mice were dissected at 20 weeks old, the livers were extracted, and liver weights per unit body weight (mg/g body weight) were determined. The results of the insulin tolerance test are shown in Table 1, the results of the oral carbohydrate tolerance test are shown in Table 2, and the results of liver weight per unit body weight are shown in Table 3.

### [Table 1]

**Table 1: Insulin tolerance test**

| | Blood sugar level after administration of insulin (mg/dl) | | | | | | | | | |
|---|---|---|---|---|---|---|---|---|---|---|
| | 0 minute | | 30 minutes | | 60 minutes | | 90 minutes | | 120 minutes | |
| | Average | S.E. | Average | S.E. | Average | S.E. | Average | S.E. | Average | S.E. |
| Reference group | 116.5 | 17.7 | 59.8 | 4.5 | 55.3 | 1.9 | 51. 8 | 1.6 | 62.0 | 2.3 |
| Group A | 210.1 | 12.6 | 87.3 | 10.7 | 83.4 | 11.2 | 115.3 | 18.5 | 136.5 | 16.6 |
| Group B | 183.6 | 8.3 | 61.5 | 3.2 | 58.6 | 3.6 | 66.8 | 4.9 | 88.5 | 8.1 |
| Group C | 194.9 | 6.0 | 88.3 | 7.4 | 85.8 | 7.5 | 102.0 | 10.3 | 135.6 | 11.7 |
| Group D | 184.5 | 8.1 | 70.8 | 5.1 | 61.8 | 4.3 | 74.9 | 6.3 | 96.0 | 7.4 |

### [Table 2]

**Table 2: Oral carbohydrate tolerance test**

| Blood sugar level after administration of glucose (mg/dl) | | | | | | | | | | |
|---|---|---|---|---|---|---|---|---|---|---|
| | 0 minute | | 30 minutes | | 60 minutes | | 90 minutes | | 120 minutes | |
| | Average | S.E. | Average | S.E. | Average | S.E. | Average | S.E. | Average | S.E. |
| Reference group | 54.5 | 1.0 | 269.3 | 24.4 | 214.8 | 19.0 | 195.3 | 9.1 | 157.5 | 20.2 |
| Group A | 125.8 | 7.7 | 440.4 | 13.4 | 424.8 | 32.1 | 424.6 | 35.7 | 357.7 | 21.6 |
| Group B | 101.5 | 10.2 | 398.9 | 20.3 | 424.1 | 23.1 | 387.9 | 21.8 | 324.0 | 21.4 |
| Group C | 141.2 | 12.2 | 450.0 | 12.8 | 481.0 | 14.8 | 445.1 | 18.4 | 343.9 | 21.9 |
| Group D | 111.4 | 8.5 | 402.6 | 21.6 | 415.7 | 26.1 | 364.8 | 28.9 | 277.3 | 21.3 |

### [Table 3]

**Table 3: Liver weight per unit body weight**

| Liver weight per unit body weight (mg/g body weight) | | |
|---|---|---|
| | Average | S.E. |
| Reference group | 37.9 | 1.8 |
| Group A | 49.7 | 2.3 |
| Group B | 40.6 | 2.7 |
| Group C | 46.6 | 2.4 |
| Group D | 41.4 | 2.6 |

As shown in Table 1, the degree of the decrease in the blood sugar level provided by the insulin administration was decreased in the group A compared with the reference group, and thus insulin resistance was observed. On the other hand, the degree of the decrease in the blood sugar level provided by the insulin administration was larger in the group B and the group D compared with the group A, and thus the insulin resistance was decreased.

As shown in Table 2, in the group A, the blood sugar level was high before the glucose administration (0 minute) compared with the reference group, the high value of the blood sugar level increased by the oral administration of glucose was maintained over 120 minutes after the administration, and thus abnormal glucose tolerance was observed. On the other hand, in the group B and the group D, the blood sugar level before the glucose administration was lower compared with the group A, the blood sugar level began to decrease after 90 minutes from the glucose administration, and thus abnormal glucose tolerance was decreased. The degree of the decrease was larger in the group D.

As shown in Table 3, in the group A, the liver weight per unit body weight was markedly larger than that of the reference group, but the liver weights per unit body weight observed for the group B and the group D were equivalent to that of the reference group. It is considered to be because hypertrophy of the liver was suppressed.

### [Example 2]

The experiment was performed in the same manner as that of Example 1 until the mice became 20 days old after the birth, then about 20 mg of feces were collected from each of the mice of the groups A to D, and crushed by using a beads crusher (Yasui Kikai Corporation), and cell DNA was extracted by the phenol/chloroform method. Real-time PCR was performed by using the obtained DNA of the intestinal bacteria as the template. By using SYBR Premix Ex Taq (Takara Bio Inc.) as the reaction reagent, and 7500 Fast Real-Time PCR System (Thermo Fisher Scientific Inc.) as the system with the primer of SEQ ID NO: 1 (ACTCCTACGGGAGGCAGCAG), and the primer of SEQ ID NO: 2 (ATTACCGCGGCTGCTGG), the total copy number of 16S rDNA per unit weight of feces was measured. In a similar manner, copy number of 16S rDNA of the *Bifidobacterium* bacterium per unit weight of feces was measured by using the primer of SEQ ID NO: 3 (CTCCTGGAAACGGGTGG), and the primer of SEQ ID NO: 4 (GGTGTTCTTCCCGATATCTACA). The ratio of the copy number of 16S rDNA of the *Bifidobacterium* bacterium to the total copy number of 16S rDNA was considered as the share (%) of the *Bifidobacterium* bacterium in the intestinal bacterial flora.

The results are shown in Table 4. Increase in the *Bifidobacterium* bacterium was observed for the group D compared with the group A, whereas increase was not observed in the group B.

[Table 4]

**Table 4: Share (%) of Bifidobacterium bacterium in intestinal bacterial flora**

| | Average | S.E. |
|---|---|---|
| Group A | 0.1 | 0.1 |
| Group B | 0.1 | 0.0 |
| Group C | 0.6 | 0.6 |
| Group D | 15.6 | 5.8 |

As shown in Tables 1, 2, and 3 mentioned above, it was found that there were observed suppression of blood sugar level elevation and no increase in liver weight per unit body weight for the groups B and D compared with the group A, and in particular, as shown in Table 2, it was found that the degree of the suppression of the blood sugar level elevation was more marked in the group D.

That is, it was found that, according to the present invention, although the effect of the present invention is exhibited regardless of the presence or absence of increase in the *Bifidobacterium* bacterium the in intestines, the effect of the present invention can be further enhanced by simultaneously taking a prebiotic as shown by the group D.

### [Preparation Example 1]

*Bifidobacterium breve* M-16V (NITE BP-02622) is added to 3 mL of the MRS liquid medium, and cultured at 37°C for 16 hours under anaerobic conditions, and then the culture liquid is concentrated and lyophilized to obtain a lyophilized powder of the bacterium (bacterial cell powder). The bacterial cell powder and a whey protein concentrate (WPC) are uniformly mixed to obtain a composition. The composition (20 g) is dissolved in water (200 g) to obtain a composition for infants and/or children for preventing a disease resulting from high blood sugar level in later childhood and thereafter. By administering the composition to an infant and/or child, a disease resulting from high blood sugar level can be prevented in the infant and/or child in later childhood and thereafter. Blood sugar level elevation can also be suppressed in the infant and/or child in later childhood and thereafter.

### [Preparation Example 2]

*Bifidobacterium breve* M-16V (NITE BP-02622) is added to 3 mL of the MRS liquid medium, and cultured at 37°C for 16 hours under anaerobic conditions, and then the culture liquid is concentrated and lyophilized to obtain a lyophilized powder of the bacterium (bacterial cell powder). The bacterial cell powder and dry powder of milk protein concentrate (MPC480, Fonterra Cooperative Group, protein content is 80%, casein protein:whey protein ratio is about 8:2) are uniformly mixed to obtain a composition. This composition (20 g) is dissolved in water (200 g) to obtain a composition for infants and/or children for preventing a disease resulting from high blood sugar level in later childhood and thereafter. By administering the composition to an infant and/or child, a disease resulting from high blood sugar level can be prevented in the infant and/or child in later childhood and thereafter. Blood sugar level elevation can also be suppressed in the infant and/or child in later childhood and thereafter.

### [Preparation Example 3]

*Bifidobacterium breve* M-16V (NITE BP-02622) is added to 3 mL of the MRS liquid medium, and cultured at 37°C for 16 hours under anaerobic conditions, and then the culture liquid is concentrated and lyophilized to obtain a lyophilized powder of the bacterium (bacterial cell powder). Then, crystalline cellulose is put into a stirring granulation machine, and mixed with the bacterial cell powder. Then, purified water is added, granulation is performed, and the granules are dried to obtain granules containing the extracted ingredients of the bacterium, prebiotics, and an excipient. By administering the granules to an infant and/or child, a disease resulting from high blood sugar level can be prevented in the infant and/or child in later childhood and thereafter. Blood sugar level elevation can also be suppressed in the infant and/or child in later childhood and thereafter.

### [Preparation Example 4]

A method for producing fermented milk containing *Bifidobacterium breve* M-16V (NITE BP-02622) is shown below.

First, a milk raw material, water as required, other ingredients, and so forth are mixed, preferably subjected to homogenization, and subjected to a heat sterilization treatment. The homogenization and heat sterilization treatment can be performed by conventional methods. A lactic starter is added (inoculated) to the heat-sterilized sterilized milk formula, the resulting mixture is maintained at a predetermined fermentation temperature to allow fermentation, and thereby obtain a fermentation product. By the fermentation, curd is formed.

As the lactic starter, for example, lactic acid bacteria usually used for production of yogurt such as *Lactobacillus bulgaricus, Lactococcus lactis,* and *Streptococcus thermophilus* can be used. When pH reaches a targeted level, the formed curd is crushed by stirring, and cooled to a temperature of 10°C or lower to obtain a fermentation product. By cooling to 10°C or lower, the activity of the lactic acid bacterium can be reduced, and generation of an acid can be thereby suppressed.

Subsequently, the fermentation product obtained by the fermentation process is subjected to a heat treatment to obtain a heated fermentation product (fermentation product after the heat treatment). By appropriately heating the fermentation product, generation of acid by the lactic acid bacterium in the heated fermentation product can be suppressed. Decrease of pH during the subsequent production process and/or storage of the concentrated fermented milk containing the bifidobacterium can be thereby suppressed, and the survivability of the bifidobacterium can be improved as a result.

Then, *Bifidobacterium breve* M-16V (NITE BP-02622) is added to the heated fermentation product obtained through the heat treatment step. Addition amount of *Bifidobacterium breve* M-16V (NITE BP-02622) is preferably 1 x 10⁷ to 1 x 10¹¹ cfu/ml, more preferably 1 x 10⁸ to 1 x 10¹⁰ cfu/ml, based on the volume of the heated fermentation product. When *Bifidobacterium breve* M-16V (NITE BP-02622) consists of dead cells, the unit of cfu/ml can be read as number of cells/ml.

After the *Bifidobacterium breve* NITE BP-02622 is added to the heated fermentation product, the mixture is concentrated. The concentration operation can be performed by using a known concentration method as required. For example, centrifugation method or membrane separation method can be used.

By the centrifugation method, whey in the mixture to be concentrated (heated fermentation product containing the bifidobacterium) is removed, and a concentrated fermented milk containing bifidobacteria in which solid concentration is increased is obtained.

By administering the fermented milk obtained as described above to an infant and/or child, a disease resulting from high blood sugar level can be prevented in the infant and/or child in later childhood and thereafter. Blood sugar level elevation can also be suppressed in the infant and/or child in later childhood and thereafter.

### [Preparation Example 5]

A method for producing a formulated powdered milk containing *Bifidobacterium breve* M-16V (NITE BP-02622) is shown below.

Desalted cow's milk serum protein powder (10 kg, Milei GmbH), milk casein powder (6 kg, Fonterra Co-operative Group), lactose (48 kg, Milei GmbH), mineral mixture (920 g, Tomita Pharmaceutical Co., Ltd.), and vitamin mixture (32 g, Tanabe Seiyaku Co., Ltd.) are dissolved in warm water (300 kg), and further dissolved for 10 minutes by heating at 90°C, a formulated fat (28 kg, Taiyo-yushi Co. Ltd.) is added to the solution, and the resulting mixture is homogenized. Then, the mixture is sterilized, concentrated, and spray-dried to prepare about 95 kg of formulated powdered milk. To this powdered milk, cell powder of *Bifidobacterium breve* M-16V (NITE BP-02622) (100 g, 1.8 x 10¹¹ cfu/g, Morinaga Milk Industry Co., Ltd.) diluted in starch is added to prepare a bifidobacterium and oligosaccharide-containing formulated powdered milk (95 kg). If the obtained formulated powdered milk is dissolved in water to obtain a formulated solution having a total solid content of 14 % (w/v), which is the standard concentration in formulated milk, number of the bifidobacteria in the formulated solution is 2.7 x 10⁹ cfu/100 ml. By administering the formulated powdered milk obtained as described above to an infant and/or child, a disease resulting from high blood sugar level can be prevented in the infant and/or child in later childhood and thereafter. Blood sugar level elevation can also be suppressed in the infant and/or child in later childhood and thereafter.

## Claims

1. A composition for infants and/or children for preventing a disease resulting from high blood sugar level in later childhood and thereafter, which contains a *Bifidobacterium* bacterium as an active ingredient.

2. The composition according to claim 1, wherein:
the disease resulting from high blood sugar level is hyperglycemia, hyperinsulinaemia, hyperlipidemia, diabetes, diabetic retinopathy, diabetic nephropathy, arteriosclerosis, myocardial infarction, non-alcoholic fatty liver, non-alcoholic steatohepatitis, liver cirrhosis, or liver cancer, and
the liver cirrhosis and liver cancer are cirrhosis and liver cancer resulting from non-alcoholic fatty liver and/or non-alcoholic steatohepatitis.

3. A composition for infants and/or children for use in suppressing blood sugar level elevation in later childhood and thereafter, which contains a *Bifidobacterium* bacterium as an active ingredient.

4. The composition according to any one of claims 1 to 3, wherein the infants and/or children are infants and/or children in a period of from birth to completion of weaning.

5. The composition according to any one of claims 1 to 4, which does not aim at increase of the *Bifidobacterium* bacterium in the intestines.

6. The composition according to any one of claims 1 to 5, wherein the *Bifidobacterium* bacterium is *Bifidobacterium breve.*

7. The composition according to claim 6, wherein the *Bifidobacterium breve* is *Bifidobacterium breve* M-16V (NITE BP-02622) .

8. The composition according to any one of claims 1 to 7, which is a food or drink composition.

9. The composition according to any one of claims 1 to 7, which is a pharmaceutical composition.

10. Use of a *Bifidobacterium* bacterium in manufacture of a composition for infants and/or children for preventing a disease resulting from high blood sugar level in later childhood and thereafter.

11. A *Bifidobacterium* bacterium, which is used for infants and/or children for preventing a disease resulting from high blood sugar level in later childhood and thereafter.

12. Use of a *Bifidobacterium* bacterium for infants and/or children for preventing a disease resulting from high blood sugar level in later childhood and thereafter.

13. A method for preventing a disease resulting from high blood sugar level in later childhood of an infant and/or child and thereafter, which comprises the step of administering a *Bifidobacterium* bacterium to the infant and/or child.
